# EUROPEAN PATENT SPECIFICATION

(11) **EP 2 643 003 B1**
(45) Date of publication and mention of the grant of the patent: **17.12.2014**
(21) Application number: 12724719.5
(22) Date of filing: 27.04.2012
(51) Int. Cl.: A61K 31/70, A61K 31/20, A61K 31/23, A61P 15/02, A61P 31/04, A61K 45/06, A61K 9/00, A61K 47/10, A61K 9/06, A61K 47/38, A61K 31/7028

(54) **VAGINAL COMPOSITION BASED ON ALKYL POLYGLUCOSIDES**
VAGINALE ZUSAMMENSETZUNG AUF BASIS VON ALKYLPOLYGLUKOSIDEN
COMPOSITION VAGINALE À BASE D'ALKYLPOLYGLUCOSIDES

(30) Priority: 29.04.2011 IT MI20110715; 29.04.2011 IT MI20110716; 29.04.2011 IT MI20110717
(43) Date of publication of application: 02.10.2013
(73) Proprietor: EFFIK INTERNATIONAL S.A., 1070 Anderlecht- Bruxelles (BE)
(72) Inventor: ARDOLINO, Luca Ivan, I-20092 Cinisello Balsamo (Milano) (IT); BRUGALI, Giuseppe, I-20092 Cinisello Balsamo (Milano) (IT)
(74) Representative: Long, Giorgio
(86) International application number: PCT/IB2012/052119
(87) International publication number: WO 2012/147056

(56) References cited:
- EP-A1- 1 609 464
- WO-A1-97/45101
- US-B1- 6 531 435
- US-B1- 6 599 521

## Description

### Field of the invention

The present invention regards a vaginal composition based on alkyl glucosides or alkyl polyglucosides, in particular for the treatment of *Streptococcus agalactiae* infections and other pathogens.

### Prior art

Group B streptococcus or *Streptococcus agalactiae* (GBS), is the aetiological agent of the severe neonatal infections in industrialised countries and it occurs in 15-35% of pregnant women. According to Blond et al. (1), the analysis of 8 published studies revealed that GBS was observed in mothers from 7.6 to 22.8% of the pregnancies. Such percentage varies depending on the ethnicities and collection sites which can be the vagina alone or the vagina and the rectum. The asymptomatic vaginal presence of GBS varies during pregnancy. The presence at vaginal level can be associated with vaginitis, urinary infections and it increases the risk of chorioamnionitis. After delivery, the consequences of the GBS infection may vary, ranging from chorioamnionitis and postpartum endometritis, to bacteremia and septicemia. Infection in the pregnant woman may also lead to early delivery, early breakage of the membranes and low weight of the newborn at birth. The infection in the contaminated newborn may also cause septicemia accompanied by shock, pneumonia, acute respiratory distress syndrome and neurological infections such as meningitis which may lead to permanent handicap, or even death.

The antibiotic treatment of the asymptomatic infection during pregnancy is not recommended. Pregnant women who are asymptomatic carriers of GBS should not be treated before labour given that the antibiotic treatment does not reduce the level of bacteria observed during labour. Furthermore, a typical problem related to the administration of antibiotics lies in the occurrence of resistance phenomena which can jeopardize the efficiency of the treatment during delivery.

Therefore, the normal therapy is that of treating - using antibiotics through intravenous administration - the woman during delivery, which however does not guarantee the total elimination of risks on the unborn baby.

The number of infected newborns ranges between 3 and 12% of the pregnancies.

Public domain data indicate that every year in the USA 12000 newborns are infected and about 2000 die.

Also the bacterial vaginosis (BV) is a vaginal infection which affects pregnant women. BV is characterised by a deep modification of the normal vaginal

flora with disappearance of *Lactobacilli* and abnormal development of a multiform flora, among which *Gardnerella vaginalis, Atopobium vaginae* and anaerobic microorganisms.

BV may cause spontaneous abortion and premature birth and it is associated to an increased risk of contracting HIV. All BV cases should be treated during pregnancy.

The antibiotic treatment of BV is not sufficient to eliminate the infection even in this case.

BV and GBS are high sources of risk that influence the result of the pregnancy, both for the newborn and for the mother.

US 6 531 435 B1 and US 6 599 521 B1 disclose a vaginal cleansing composition for inhibiting the production of exoprotein grom Gram positive bacteria comprising 0.25-5 mmol of an alkyl polyglucoside having 8-14 C atoms.

EP 1 609464 A1 discloses a composition for cleaning vaginal mucous membranes comprising alkyl glucoside (lauryl and/or decyl clucoside) and middle chian saturated fatty acids or the glyceride ester derivatives thereof.

WO 97/45101 A1 discloses the use of alpha-alkylglucosides as antibacterial agents against *Candida albicans,* but not in vaginal compositions.

### Summary of the invention

Therefore, an object of the present invention is that of providing a treatment for the vaginal infections that is safe and efficient, so as to be proposed both for the treatment of the asymptomatic infections and the symptomatic ones also at an early stage of pregnancy.

Such treatment is conducted, according to the invention, by means of a bactericidal or bacteriostatic agent, alone or combined with other active ingredients. The bactericidal or bacteriostatic agent of the invention belongs to the class of the alkyl glucosides or the alkyl polyglucosides.

In an embodiment, the treatment according to the invention aims at preventing and treating *Streptococcus agalactiae* infections.

Thus, a bactericidal or bacteriostatic vaginal formulation containing one or more active ingredients according to the invention, among which at least one is selected in the class of the alkyl glucosides or the alkyl polyglucosides, alongside pharmaceutically acceptable excipients and carriers forms another object of the invention.

Particular objects of the invention are those mentioned in the attached claims, whose definitions are an integral part of the present description.

### Detailed description of the invention

The present invention aims at providing a compound belonging to the class of the alkyl glucosides or the alkyl polyglucosides for use in the prevention and in the treatment of vaginal infections caused by *Streptococcus agalactiae* and by other pathogens.

The alkyl glucosides or alkyl polyglucosides are non-ionic surfactants which derive from the reaction of starch with a fatty alcohol. Examples of alkyl glucosides or alkyl polyglucosides which can be used for the objects of the invention are: decyl glucoside, caprylyl/capryl glucoside, lauryl glucoside, coco glucoside, C8-C10 alkyl polyglucoside, C12-C16 alkyl polyglucoside. Such substances are commonly available in the market.

In a preferred embodiment, caprylyl/capryl glucoside, lauryl glucoside, C8-C10 alkyl polyglucoside, C12-C16 alkyl polyglucoside or mixtures thereof will be used.

The invention also regards a compound belonging to the class of the alkyl glucosides or alkyl polyglucosides in association with an active ingredient selected within the category of the middle-chain saturated fatty acids or the glycerol ester derivatives thereof and relative mixtures for use as a bacteriaostatic or bactericidal agents in the prevention and in the treatment of the bacterial infections of the vaginal tract. Such bacterial infections of the vaginal tract are in particular *Streptococcus agalactiae* infections.

Examples of middle-chain saturated fatty acids or the glycerol ester derivatives thereof which can be used for the subject of the invention are: lauric acid, capric acid, caprylic acid and caproic acid and the glycerol ester derivatives thereof. These substances are commonly available in the market. Lauric acid and monolaurate (monoglycerol ester of lauric acid) and mixtures thereof may preferably be used in the formulate. Common natural sources of lauric acid are cocoa oil or palm shell oil and they can be used for the object of this invention.

The use of an alkyl glucoside or alkyl polyglucoside in association with lauric acid or monolaurate according to the invention comprises both the presence of active ingredients in the same composition and the separate, simultaneous or differed use of the various active ingredients.

The invention also regards a compound selected from among caprylyl/capryl glucoside, lauryl glucoside, C8-C10 alkyl polyglucoside, C12-C16 alkyl polyglucoside and mixtures thereof for use in the prevention and in the treatment of bacterial infections of the vaginal tract.

The term "bacterial infections of the vaginal tract" comprises *Gardnerella vaginalis, Candida albicans, Neisseria gonorrheae, Atopobium vaginae, Chlamidia trachomatis, Trichomonas vaginalis, Mycoplasma genitalium*/*urealiticum*/*hominis*/*parvum, Treponema pallidum* and *Streptococcus agalactiae* infections.

A further object of the invention is that of providing a bactericidal and/or bacteriostatic vaginal formulation comprising at least one compound belonging to the class of the alkyl glucosides or alkyl polyglucosides, as defined above, possibly in association with an active ingredient selected from among lauric acid, monolaurate and mixtures thereof.

The alkyl glucoside, alkyl polyglucoside, C8-C10 alkyl polyglucoside, C12 C16 alkyl polyglucoside compound and the further active ingredient selected from among lauric acid, monolaurate and mixtures thereof are preferably at a ratio comprised between 1:10 and 10:1.

### Evaluation of the inhibitory and bactericidal activity of the compounds of the invention with respect to Streptococcus agalactiae, Gardnerella vaginalis, Neisseria gonorrhoeae and Candida albicans

The experiments were carried out using two different alkyl glucosides, i.e.: caprylyl/capryl glucoside/C8-C10 alkyl polyglucoside CASR-No. 68515-73- 1 (A1) and lauryl glucoside/C12-C16 alkyl polyglucoside CASR-No. 110615-47-9 (A2).

Two different active ingredients, lauric acid (F) and monolaurate (G) were also tested both alone and combined with an alkyl glucoside.

By comparison, the lactobacillus strains dominating in the physiological colonization of the vaginal mucosa typical in healthy women were also tested.

It is known that the three strains dominating the colonization of the vaginal mucosa in healthy women are: *Lactobacillus crispatus, Lactobacillus jensenii* and *Lactobacillus gasseri.*

Thus, such explorative activity was carried out on such strains with the aim of evaluating a selectivity of the compounds of the invention with respect to the pathogenic microorganisms with respect to the lactobacillus bacterial flora.

### BACTERIAL STRAINS

Three *Streptococcus agalactiae* strains isolated from vaginal-rectal buffers during the prenatal check-ups were used for the assay. The strains were named "strain 2", "strain 10" and "strain 11". Isolation was carried out on a blood agar medium and identification was obtained through biochemical tests of the API 20 Strep (Bio-Merieux) system and through the identification of the Lancefield group.

The experiments were also carried out on a *Streptococcus agalactiae* ATCC 12386 strain.

The *Gardnerella vaginalis* ATCC 14018 strain, the *Neisseria gonorrhoeae* ATCC 43069 strain and the *Candida albicans* ATCC 10231 strain were also used for the experiment.

The vaginal lactobacillus strains used are the NCIMB 4505 strain of *Lactobacillus crispatus*, the NCIMB 13279 strain of *Lactobacillus jensenii* and NCIMB 702820 strain of *Lactobacillus gasseri.*

The strains were reconstituted and maintained at a temperature of -80°C in a liquid blood medium + glycerol suspension at 20%.

### CULTURE MEDIA AND SOLUTIONS

"Brain Heart Infusion broth" (BHIb, Becton Dickinson) medium was used for the *Streptococcus agalactiae* culture, "Mueller-Hinton broth" (M-Hb) medium after adding 2% "laked" horse blood and the "Mueller-Hinton agar" (M-Ha) medium with addition of defibrinated horse blood at 5% (Oxoid) were used for the antimicrobial activity assays.

The following table shows the media and the culture conditions of the inoculums, for determining the MIC and for determining the MBC regarding the other assayed strains.

**TABLE A**

| | ***Gardnerella vaginalis* ATCC 14018** | ***Neisseria gonorrhoeae* ATCC 43069** | ***Candida albicans* ATCC 10231** | ***Lactobacillus crsipatus*/*jensenii*/*gasseri*** |
|---|---|---|---|---|
| **Inoculum culture** | BHIB (BD*) + 2% | BHIB(BD*) + 2% Vitox | RPMI 1640-15-702 | BHIB (BD*) + 2% "laked" horse blood |
| **medium** | Horse serum (Oxoid) | (Oxoid) | (without sodium bicarbonate) (Lonza)+ 2% glucose | (Oxoid) or MRS broth |
| **Culture conditions** | 37°C + 5%CO2 x 48 hrs | 37°C + 5%CO2 x 48 hrs | 35°C x 24 hrs | 37° C + 5% CO2 x 48 hrs |
| **Culture medium for determining MIC** | BHIB (BD*) + 2% Horse serum (Oxoid) | BHIB (BD*) + 2% Vitox (Oxoid) | RPMI 1690-15-702 (without sodium bicarbonate) (Lonza)+ 2% glucose | BHIB (BD*) + 2% Horse serum (Oxoid) or MRS broth |
| **Culture medium for determining MBC** | BHI agar (BD*) + 7% blood, heated ("chocolate agar" | BHI agar (BD*) + 7% blood, heated ("chocolate agar") | Sabouraud dextrose agar (Oxoid) | M-Ha +5% defibrinated horse blood (Oxoid) or "Rogosa agar" (Oxoid) |

| | | | | |
|---|---|---|---|---|
| *Becton-Dickinson | | | | |

### PREPARATION OF THE BACTERIAL INOCULUM

The *Streptococcus agalactiae* strains were cultured in BHIb broth for 24 hours at 37°C. Immediately before the assay, the bacterial suspensions were diluted up to obtaining turbidity equivalent to 0.5 McFarland standard. 50 µl of a further 1:100 dilution in broth + blood at 2% were distributed in the wells of the microtitre plates. The presumed titre of the inoculum carried out through this procedure is of about 5x10⁴ ufc (units forming colony)/well.

The bacterial cultures were also diluted serially according to a value 10 (up to a dilution value equivalent to 10⁻⁷) and an aliquot of 0.1 ml of each dilution was double streaked on M-Ha + blood 5% for determining the actual titre to be used, subsequently, to determine the MBC (Minimum Bactericidal Concentration).

The other assayed microbial strains were cultured in the liquid media and in the incubation conditions specified in table A, following the operating procedure described above.

### PREPARATION OF THE DILUTIONS OF THE ANALYSED PRODUCT

The substances to be subjected to the analysis were prepared for the assay through dilution and sterilisation as described hereinafter.

The concentration of the substances referred to as "mother" represents the highest concentration at which complete solubilisation could be obtained and it is 4 times higher than the highest concentration assayed in the test.

| **Substance** | **solvent** | **"mother" conc.** |
|---|---|---|
| A1 | water | 100.16 mg/ml |
| A2 | water | 48.4 mg/ml |
| F | prop glycol 30% in water | 10.08 mg/ml |
| G | prop glycol 50% in water | 34.7 mg/ml |
| A1+F | water prop glycol 3.12-2.48 mg/ml 25% in water | |
| A1+G | prop glycol 3.12-1.09 mg/ml 25% in water | |
| A2+F | prop glycol 0.76-2.48 mg/ml 25% in water | |
| A2+G | prop glycol 0.76-1.09 mg/ml 25% in water | |

### DETERMINATION OF THE MINIMUM INHIBITORY CONCENTRATION (MIC)

### Streptococcus agalactiae

The assay was carried out in 96-well microtitre plates. 50 µl of M-Hb at normal concentration were deposited in the first well of each of the 5 rows. Starting from the first well of each row, 50 µl volumes were transferred from each well to the subsequent one 10 times, thus obtaining a series of dilutions after doubling. The 12^{th} well of each row was kept substance-free as a positive control of the bacterial growth.

Subsequently, 50 µl of bacterial inoculum of M-Hb + double concentration blood (4%) were deposited in all wells of the plate, except for the 11^{th} of each row. Only M-Hb + double concentration blood but bacteria-free, were deposited in the 11^{th} well, with the aim of providing negative control for each row. The described scheme was used for the assay of each of the three *Streptococcus agalactiae* strains.

After incubation at 37°C for 24 hours, the microtitre plates were examined to verify, in each well, the presence or absence of bacterial growth. For each substance there was determined the minimum inhibitory concentration defined as the lowest concentration capable of inhibiting bacterial growth i.e. preventing the liquid from becoming turbid within the well.

### Gardnerella vaginalis, Neisseria gonorrhoeae, Candida albicans, Lactobacillus crispatus, Lactobacillus jensenii, Lactobacillus gasseri

The assay was carried out in 96-well microtitre plates. 50 µl of substance solution at the "mother" concentration alongside 50 µl of liquid medium at double concentration were deposited in the first well of each of the 8 rows while 50 µl of liquid medium at normal concentration were deposited in the remaining 11 wells. Starting from the first well of each row, 50 µl volumes were transferred from each well to the subsequent one 10 times, thus obtaining a series of 11 dilutions after doubling. The 12^{th} well of each row was kept substance-free as a function of positive control of the bacterial growth.

Subsequently, 50 µl of diluted bacterial inoculum as specified previously were deposited in all wells of the plate, except for the 11^{th} of each row. Only the liquid medium at single concentration but bacteria-free, was deposited in the 11^{th} well, with the aim of providing negative control for each row. The described scheme was used for the assay with each of the assayed microbial strains.

After incubation at the conditions indicated regarding each micro-organism, the microtitre plates were examined to verify, in each well, the presence or absence of bacterial growth. The minimum inhibitory concentration is defined as the lowest concentration of the substance still capable of inhibiting bacterial growth i.e. preventing the liquid from becoming turbid within the well.

### DETERMINATION OF THE MINIMUM BACTERICIDAL CONCENTRATION (MBC)

Immediately after determining the value of the MIC for each substance, a volume equivalent to 50 µl was taken from each well and streaked on the surface of plates containing the solid medium. The plates were subsequently incubated at the conditions indicated for each microbial strain. After incubation at 37°C for 24 hours, the number of colonies grown on the medium surface was counted thus determining the number of bacteria that survived after 24 hours of contact with the substance at the concentration present in the well. The MBC value defined as the lowest concentration of each substance capable of reducing the bacterial load by 99.9% in the previously defined assay conditions was determined through comparison between the number of bacteria that survived in each well and that of the bacterial inoculum deposited initially.

### DETERMINATION OF THE MINIMUM INHIBITORY CONCENTRATION (MIC) ON AGARISED MEDIUM

The assay was carried out on an M-Ha medium + blood at 5%. The medium was prepared at a concentration 33% higher with respect to the final one used in the test corresponding to the one indicated by the supplier. After sterilisation in autoclave, the medium was balanced at the temperature of 48° C and added with horse blood at the concentration of 6.6% (33% higher than the final concentration of the assay). 15 ml aliquots of M-Ha medium + blood were transferred into 50 ml falcon test tubes maintained at 48° C, added with 5 ml of the dilutions of the substances to be assayed, mixed, poured into Petri dishes and left to solidify. Twenty-five bacterial inoculums, each with approximate volume of about 5 µl and containing about 10⁵ ufc, were deposited on the surface of the M-Ha medium + blood of each plate. After incubation at 37° C for 18 hours the minimum inhibitory concentration (MIC) defined as the minimum concentration of substance capable of inhibiting a bacterial growth noticeable to the naked eye at the area of deposition of the inoculums was read.

### RESULTS

In the test with *Streptococcus agalactiae* the minimum inhibitory concentrations in the assay in liquid medium, regarding which only the bactericide minimum concentration values are indicated, could not be determined with sufficient reliability due to the turbidity of the solutions containing the substances. "Determination of the Minimum Inhibitory Concentration on Agarised Medium" was carried out to obtain the MIC values.

Table I shows the bacterial concentration values of the cultures used for preparing the inoculums and the concentrations of the suspensions of the inoculums.

Tables II and III show the MIC values and, respectively, the MBC values of the substances being analysed with respect to the assayed *Streptococcus agalactiae* bacterial strains.

Tables IV, V, VI show the MIC and MBC values respectively regarding *Gardnerella vaginalis, Neisseria gonorrhoeae* and *Candida albicans.*

MIC and MBC comparison data regarding substances known for their antibacterial activity with respect to assayed strains are also shown. Tables VII, VIII and IX show the MIC and MBC values respectively regarding *Lactobacillus crispatus, Lactobacillus jensenii and Lactobacillus gasseri.*

### CONCLUSIONS

The MBC could be determined for all examined substances. The MBC values regarding *S. agalactiae* seem similar among various bacterial strains. The highest values observed with strain 10 regarding substances A2 and G are due to the greater resistance revealed by strain 10 with respect to the tested antibacterial agents, in that this strain was more resistant to the reference antibiotic treatment, ampicillin. Actually, the ampicilin concentrations required to perform the bactericidal action were at least 15 times higher in the strain 10 with respect to those against strain 2 and 11 (table II). Remarks similar to those indicated regarding the MBC test also apply as regards the results of the MIC test (table III).

In conclusion, the experiments reveal that the tested molecules perform bacteriostatic and bactericidal activity both on the *Streptococcus agalactiae* ATCC strains and on the bacterial strains isolated from patients, and even more important on the ATCC strain representing serotype III responsible for at least 60% of the early infections of the newborn, also referred to as Early Onset Diseases, which are associated to the higher rate of morbidity and the higher risk of neonatal mortality.

The alkyl polyglucosides revealed high bacteriostatic and antibacterial activity both when used alone and in association with lauric acid and monolaurate.

In particular, table II shows that the association between an alkyl polyglucoside and lauric acid or monolaurate leads to a synergic effect.

**Table I - Concentration of the suspensions used as inoculum of the assayed bacterial cultures**

| **Bacterial strain** | **Inoculum suspension (cfu/ml)** |
|---|---|
| **Strain 2 *(S. agalactiae)*** | **2.5x10⁶** |
| **Strain 10 *(S. agalactiae)*** | **7x10⁶** |
| **Strain 11 *(S. agalactiae)*** | **3.85x10⁶** |
| **ATCC 12386 *(S. agalactiae)*** | **1.4x10⁶** |
| **ATCC 14018 *(G. vaginalis)*** | **4.5x10⁶** |
| **ATCC 43069 *(N*. *gonorrhoeae)*** | **7.5x10⁵** |
| **ATCC 10231 *(C. albicans)*** | **1x10⁴** |
| **NCIMB 4505 (L. crispatus)** | **5.7x10⁵** |
| **NCIMB 13279 (L. jensenii)** | **6.4x10⁵** |
| **NCIMB 702820 (L. gasseri)** | **1.58x10⁷** |

**Table II- Antibacterial activity of the substances being analysed against Streptococcus agalactiae, expressed as MBC**

| **Substance** | **Strain 11 in M-Hb blood 2%** | **Strain 10 in M-Hb blood 2%** | **Strain 2 in M-Hb blood 2%** | **ATCC 12386 in M-Hb blood 2%** |
|---|---|---|---|---|
| **A1** | 0.097 | 0.194 | 0.097 | 0.097 |
| **A2** | 0.0097 | 0.019 | 0.0097 | 0.0097 |
| **F** | 0.25 | ≥0.25 | ≥0.25 | 0.25 |
| **G** | 0.107 | 0.428 | 0.107 | 0.107 |
| **A1 + F** | - | - | - | 0.048-0.061 |
| **A1 + G** | 0.048-0.027 | >0.048-0.027 | 0.024-0.014 | 0.024-0.014 |
| **A2 + F** | - | - | - | 0.0048-0.03 |
| **A2 + G** | >0.0097/ 0.027 | 0.0048-0.013 | 0.0048-0.013 | 0.0048/0.013 |
| **Ampicillin (µg/ml)** | <0.06 | 1 | <0.06 | 0.5 |

| | | | | |
|---|---|---|---|---|
| A1 = Caprylyl/Capryl Glucoside, C8-C10 alkyl polyglucoside (CASR-no. 68515-73-1) A2 = Lauryl Glucoside, alkyl polyglucoside C12-C16 (CASR-no. 110615-47-9) F = lauric acid G = monolaurate (monoglycerol ester of lauric acid) | | | | |

The two MBC values indicated in table II for A1+F, A1+G, A2+F and A2+G respectively regard the first and the second active ingredient used combined.

**Table III - Inhibitory activity of the substances being analysed against Streptococcus agalactiae, expressed as MIC on AGARISED medium**

| MIC (minimum inhibiting concentration) expressed in % | | | | | | | | | | | | |
|---|---|---|---|---|---|---|---|---|---|---|---|---|
| | A1 | A1F | | A1G | | A2 | A2G | | A2F | | G | F |
| | | | | | | | | | | | | |
| 5 | 0,093 | 0,024 | 0,03 | 0,024 | 0,014 | 0,019 | 0,0048 | 0,014 | 0,0048 | 0,03 | 0,11 | 0,25 |
| 6 | 0,048 | 0,024 | 0,03 | 0,024 | 0,014 | 0,0097 | 0,0048 | 0,014 | 0,0048 | 0,03 | 0,11 | 0,25 |
| 7 | 0,093 | 0,024 | 0,03 | 0,024 | 0,014 | 0,0097 | 0,0048 | 0,014 | 0,0048 | 0,03 | 0,11 | 0,25 |
| 8 | 0,19 | 0,024 | 0,03 | 0,024 | 0,014 | 0,019 | 0,0048 | 0,014 | 0,0048 | 0,03 | 0,22 | 0,25 |
| 9 | 0,093 | 0,024 | 0,03 | 0,024 | 0,014 | 0,0097 | 0,0048 | 0,014 | 0,0048 | 0,03 | 0,11 | 0,25 |
| 10 | 0,19 | 0,024 | 0,03 | 0,024 | 0,014 | 0,019 | 0,0048 | 0,014 | 0,0048 | 0,03 | 0,22 | 0,25 |
| 11 | 0,093 | 0,024 | 0,03 | 0,024 | 0,014 | 0,0097 | 0,0048 | 0,014 | 0,0048 | 0,03 | 0,11 | 0,25 |
| 12 | 0,093 | 0,024 | 0,03 | 0,024 | 0,014 | 0,0097 | 0,0048 | 0,014 | 0,0048 | 0,03 | 0,11 | 0,25 |
| 13 | 0,093 | 0,024 | 0,03 | 0,024 | 0,014 | 0,0097 | 0,0048 | 0,014 | 0,0048 | 0,03 | 0,11 | 0,25 |
| 14 | 0,093 | 0,024 | 0,03 | 0,024 | 0,014 | 0,0097 | 0,0048 | 0,014 | 0,0048 | 0,03 | 0,11 | 0,25 |
| 15 | 0,048 | 0,024 | 0,03 | 0,024 | 0,014 | 0,019 | 0,0048 | 0,014 | 0,0048 | 0,03 | 0,11 | 0,25 |
| 16 | 0,093 | 0,024 | 0,03 | 0,024 | 0,014 | 0,0097 | 0,0048 | 0,014 | 0,0048 | 0,03 | 0,11 | 0,25 |
| 17 | 0,048 | 0,024 | 0,03 | 0,024 | 0,014 | 0,0097 | 0,0048 | 0,014 | 0,0048 | 0,03 | 0,11 | 0,25 |
| 18 | 0,19 | 0,024 | 0,03 | 0,024 | 0,014 | 0,019 | 0,0048 | 0,014 | 0,0048 | 0,03 | 0,22 | 0,25 |
| 19 | 0,093 | 0,024 | 0,03 | 0,024 | 0,014 | 0,0097 | 0,0048 | 0,014 | 0,0048 | 0,03 | 0,11 | 0,25 |
| 2 | 0,093 | 0,024 | 0,03 | 0,024 | 0,014 | 0,0097 | 0,0048 | 0,014 | 0,0048 | 0,03 | 0,11 | 0,25 |
| 12386 | 0,093 | 0,024 | 0,03 | 0,024 | 0,014 | 0,0097 | 0,0048 | 0,014 | 0,0048 | 0,03 | 0,11 | 0,25 |
| 12403 | 0,093 | 0,024 | 0,03 | 0,024 | 0,014 | 0,0097 | 0,0048 | 0,014 | 0,0048 | 0,03 | 0,11 | 0,25 |
| 27956 | 0,093 | 0,024 | 0,03 | 0,024 | 0,014 | 0,019 | 0,0048 | 0,014 | 0,0048 | 0,03 | 0,11 | 0,25 |
| 20 | 0,048 | 0,024 | 0,03 | 0,024 | 0,014 | 0,0097 | 0,0048 | 0,014 | 0,0048 | 0,03 | 0,11 | 0,25 |
| 21 | 0,048 | 0,024 | 0,03 | 0,024 | 0,014 | 0,0097 | 0,0048 | 0,007 | 0,0048 | 0,03 | 0,11 | 0,25 |
| 22 | 0,093 | 0,024 | 0,03 | 0,024 | 0,014 | 0,0097 | 0,0048 | 0,014 | 0,0048 | 0,03 | 0,11 | 0,25 |
| 23 | 0,093 | 0,024 | 0,03 | 0,024 | 0,014 | 0,0097 | 0,0048 | 0,014 | 0,0048 | 0,03 | 0,11 | 0,25 |
| 24 | 0,093 | 0,024 | 0,03 | 0,024 | 0,014 | 0,0097 | 0,0048 | 0,014 | 0,0048 | 0,03 | 0,11 | 0,25 |
| 25 | 0,048 | 0,024 | 0,03 | 0,024 | 0,014 | 0,0097 | 0,0048 | 0,014 | 0,0048 | 0,03 | 0,11 | 0,25 |

Also regarding the other assayed bacterial strains, there was revealed a considerable antibacterial and bacteriostatic activity of the alkyl polyglycosides subject of the invention.

A significant synergic effect of the alkyl polyglucosides both with lauric acid and with monolaurate was observed in all cases.
Key:
A2 = Lauryl Glucoside, C12-C16 alkyl polyglucoside (CASR-no. 110615-47-9)
F = lauric acid
G = monolaurate (monoglycerol ester of lauric acid)

**Table IV - MIC and MBC against Gardnerella vaginalis**

| | **MIC (%)** | **MBC (%)** |
|---|---|---|
| A2 | 0.0048 | 0.0048 |
| A2 + F | 0.00008/0.00047 | 0.0003/0.0019 |
| A2 + G | 0.00015/0.00042 | 0.0003/0.00083 |
| metronidazole | 0.0004 | 0.0008 |

**Table V - MIC and MBC against Neisseria gonorrhoeae**

| | **MIC (%)** | **MBC (%)** |
|---|---|---|
| A2 | ≤0.0012 | ≤0.0012 |
| A2 + F | ≤0.00004/0.00024 | ≤0.00004/0.00024 |
| A2 + G | ≤0.00004/0.00011 | ≤0.00004/0.00011 |
| ampicillin | ≤0.000012 | ≤0.000012 |

**Table VI - MIC and MBC against Candida albicans**

| | **MIC (%)** | **MBC (%)** |
|---|---|---|
| A2 | 0.077 | 0.154 |
| A2 + F | 0.0048/0.03038 | 0.00969/0.06076 |
| A2 + G | 0.0048/0.0132 | 0.0048/0.0132 |
| econazole | 0.000025 | 0.0008 |
| miconazole | 0.000012 | 0.0008 |

Regarding the tested vaginal lactobacilli there was observed a marginal antibacterial and bacteriostatic activity, as shown in Tables VII, VIII and IX.

There emerged the following interesting situation:
- regarding substances A2, A1+G, A2+G and A1 there were observed MBC values ranging between 2 and 8 times higher than those observed in the *S. agalactiae* strains;
- regarding substances A2, A2+F and A2+G there were observed MBC values about 15 times higher than those observed in G. *vaginalis and* ranging between 60 and 120 times higher than those observed in *N. gonorrhoeae*.

All this to the advantage of efficacy against the tested pathogen strains (excluding the lactobacilli), though maintaining a neutral action with respect to vaginal lactobacilli.

**Table VII - MIC and MBC against Lactobacillus crispatus**

| | **MIC (%)** | **MBC (%)** |
|---|---|---|
| A1 | 0.097 | 0.193 |
| A1 + F | 0.012/0.0152 | 0.0242/0.0304 |
| A1 + G | 0.0242/0.0136 | 0.0484/0.0272 |
| A2 | 0.038 | 0.077 |
| A2 + F | 0.0024/0.0152 | 0.0048/0.0304 |
| A2 + G | 0.0048/0.0136 | 0.0096/0.0272 |

**Table VIII - MIC and MBC against Lactobacillus jensenii**

| | **MIC (%)** | **MBC (%)** |
|---|---|---|
| A1 | 0.193 | 0.193 |
| A1 + F | 0.0242/0.0304 | 0.0242/0.0304 |
| A1 + G | 0.0242/0.0136 | 0.0242/0.0136 |
| A2 | 0.077 | 0.077 |
| A2 + F | 0.0048/0.0304 | 0.0048/0.0304 |
| A2 + G | 0.0048/0.0136 | 0.0048/0.0136 |

**Table IX - MIC and MBC against Lactobacillus gasseri**

| | **MIC (%)** | **MBC (%)** |
|---|---|---|
| A1 | 0.097 | 0.193 |
| A1 + F | 0.0242/0.0304 | 0.0484/0.06076 |
| A1 + G | 0.0484/0.026 | 0.0968/0.0544 |
| A2 | 0.077 | 0.077 |
| A2 + F | 0.0048/0.0304 | 0.0048/0.0304 |
| A2 + G | 0.0096/0.026 | 0.0192/0.0544 |

According to the present invention the dosage of compounds proposed for administration to a woman (with about 70 Kg body weight) ranges from 0.01 mg to 1 g and, preferably, from 0.1 mg to 100 mg of the active ingredient per dose unit. The dosage unit can be administered, for example, from 1 to 4 times a day. It should be considered that continuous dosage variations may be required depending on the seriousness of the clinical conditions to be treated. The exact dosage is at the discretion of the doctor.

The treatment according to the invention may comprise the topic administration of formulations containing the abovementioned compounds starting from the 32^{nd} or from the 35^{th} week of pregnancy up to delivery or, if necessary, even after. Actually, the compounds of the invention have a low toxicity and do not give rise to resistance phenomena.

The bactericidal and/or bacteriostatic vaginal formulations according to the invention can for example be in form of a vaginal gel, a vaginal lavage, a vaginal cream, ointment, vaginal foam, vaginal tablets, hard and soft vaginal capsules. The pharmaceutical forms previously mentioned herein can be released immediately or through a modified release depending on the need.

The introduction of the dosage unit into the vaginal cavity may be facilitated by the use of specific suitable techniques (vaginal applicators, syringes, wads etc...).

A solvent is normally provided for in order to facilitate the incorporation of the active ingredients subject of the present invention. Though different compounds can be used for such purpose, water represents the preferred solvent due to the greater biocompatibility thereof. Also non-aqueous compounds such as glycols, for example propylene glycol, butylene glycol, ethylene glycol, hexylene glycol, polyethylene glycol, etc; and alcohols such as ethanol, propanol, isopropanol; and mixtures thereof can be used to this aim.

Typically, the solvent is present at amounts greater than about 75%, in some formulations it can be greater than about 90%; lastly, in other cases it can be comprised between about 90% and about 99.99% of the final formulate.

**EXAMPLE 1 - vaginal gel**

| **N°** | **INGREDIENT** | **TITRE** | **%** | **Var.** | **Actual Tit %** |
|---|---|---|---|---|---|
| 1 | **PURIFIED WATER** | pure | **50.265** | | |
| 2 | **PROPYLENE GLYCOL** | pure | **40.000** | | |
| 3 | **HYDROXYPROPYL CELLULOSE** | pure | **1.300** | | |
| 4 | **A1 - CAPRYLYL-CAPRYL GLUCOSIDE** | 62.00 | **0.156** | | **0.097** |
| 6 | **GLYCERINE** | 9.00 | **6.779** | | 0.0048/0.0304 |
| 7 | **MONOHYDRATE CITRIC ACID** | pure | 1.500 | Up to pH 4.5 | 0.0048/0.0136 |
| TOTAL | | | 100.00 | | |

**EXAMPLE 2 - vaginal gel**

| **N°** | **INGREDIENT** | **TITRE** | **%** | **Var.** | **Actual Tit %** |
|---|---|---|---|---|---|
| 1 | **PURIFIED WATER** | pure | **50.305** | | |
| 2 | **PROPYLENE GLYCOL** | pure | **31.676** | | |
| 3 | **HYDROXYETHYL CELLULOSE** | pure | **1.500** | | |
| 4 | **A2 - LAURYL GLUCOSIDE** | 51.00 | **0.019** | | **0.0097** |
| 6 | **GLYCERINE** | 89.00 | **15.00** | | |
| 7 | **MONOHYDRATE CITRIC ACID** | pure | 1.500 | Up to pH 4.5 | |
| TOTAL | | | 100.00 | | |

**EXAMPLE 3 - vaginal lavage**

| **N°** | **INGREDIENT** | **TITRE** | **%** | **Var.** | **Actual Tit %** |
|---|---|---|---|---|---|
| 1 | **PURIFIED WATER** | pure | **50.316** | | |
| 2 | **PROPYLENE GLYCOL** | pure | **35.000** | | |
| 3 | **PEG 70** | 30.00 | **5.000** | | |
| 4 | **A1 - CAPRYLYL-CAPRYL GLUCOSIDE** | 62.00 | **0.078** | | **0.0484** |
| 5 | **G - MONOLAURATE** | 99.00 | **0.027** | | **0.0270** |
| 6 | **GLYCERINE** | 89.00 | **8.579** | | |
| 7 | **MONOHYDRATE CITRIC ACID** | pure | 1.000 | Up to pH 4.5 | |
| TOTAL | | | 100.00 | | |

**EXAMPLE 4 - vaginal lavage**

| **N°** | **INGREDIENT** | | **TITRE** | **%** | **Var.** | **Actual Tit %** |
|---|---|---|---|---|---|---|
| **1** | **PURIFIED WATER** | | **pure** | **53.4605** | | |
| **2** | **PROPYLENE GLYCOL** | | **pure** | **15.000** | | |
| **3** | **PEG 70** | | **30.00** | **15.000** | | |
| **4** | **A2 - LAURYL GLUCOSIDE** | | **51.00** | **0.0095** | | **0.048** |
| **5** | **F - LAURIC ACID** | | **98.00** | **0.03** | | **0.03** |
| **6** | **GLYCERINE** | | **89.00** | **15.00** | | |
| **7** | **MONOHYDRATE CITRIC ACID** | | **pure** | **1.000** | **Up to pH 4.5** | |
| **TOTAL** | | | | **100.00** | | |

### Bibliographical references:

1. Blond MH, Poulain P, Gold F, Bingen E, Watier H; Quentin R. Infection bactérienne materno-foetale. EMC 2004 Obstétrique vol 2, page 14.

## Claims

1. Compound selected from among caprylyl/capryl glucoside, lauryl glucoside, C8-C10 alkyl polyglucoside, C12-C16 alkyl polyglucoside and mixtures thereof for use as a bacteriostatic or bactericidal agent in the prevention and in the treatment of bacterial infections of the vaginal tract.

2. Compound according to claim 1 for use according to claim 1, wherein said bacterial infections of the vaginal tract comprise *Gardnerella vaginalis, Neisseria gonorrheae, Atopobium vaginae, Chlamidia trachomatis, Trichomonas vaginalis, Mycoplasma genitalium*/*urealiticum*/*hominislparvum, Treponema pallidum* and *Streptococcus agalactiae* infections.

3. Compound according to claim 1 for use according to claim 1, wherein said bacterial infections of the vaginal tract are *Streptococcus agalactiae* infections.

4. Compound according to any one of claims 1 to 3 for use according to claim 1, wherein said prevention and/or treatment provide for the administration of said compound starting from the 32^{nd} or from the 35^{th} week of pregnancy.

5. Bactericidal and/or bacteriostatic vaginal formulation for use in the prevention and in the treatment of bacterial infections of the vaginal tract, comprising at least one compound according to claim 1, possibly in association with an active ingredient selected from among lauric acid, monoglycerol ester of lauric acid and mixtures thereof.

6. Bactericidal and/or bacteriostatic vaginal formulation for the use according to claim 5, wherein said compound selected from among caprylyl/capryl glucoside, lauryl glucoside, C8-C10 alkyl polyglucoside, C12-C16 alkyl polyglucoside and mixtures thereof and said active ingredient selected from among lauric acid, monoglycerol ester of lauric acid and mixtures thereof are preferably at a ratio comprised between 1:10 and 10:1.

7. Bactericidal and/or bacteriostatic vaginal formulation for the use according to claim 5 or 6, wherein said formulation is a vaginal gel, a vaginal lavage, a vaginal cream, ointment, vaginal foam, vaginal tablets, hard and soft vaginal capsules.

## Patentansprüche

1. Verbindung, ausgewählt aus Caprylyl/Capryl-Glucosid, Lauryl-Glucosid, C8-C10-Alkyl-Polyglucosid, C12-C16-Alkyl-Polyglucosid und Mischungen davon, zur Verwendung als ein bakteriostatisches oder bakterizides Mittel in der Vorbeugung und in der Behandlung von bakteriellen Infektionen des Vaginaltrakts.

2. Verbindung nach Anspruch 1 zur Verwendung nach Anspruch 1, wobei die bakteriellen Infektionen des Vaginaltrakts *Gardnerella vaginalis, Neisseria gonorrheae, Atopobium vaginae, Chlamidia trachomatis, Trichomonas vaginalis, Mycoplasma genitalium*/*urealiticum*/*hominis*/*parvum, Treponema pallidum* und *Streptococcus agalactiae* Infektionen umfassen.

3. Verbindung nach Anspruch 1 zur Verwendung nach Anspruch 1, wobei die bakteriellen Infektionen des Vaginaltrakts *Streptococcus agalactiae* Infektionen sind.

4. Verbindung nach einem der Ansprüche 1 bis 3 zur Verwendung nach Anspruch 1, wobei die Vorbeugung und/oder Behandlung die Verabreichung der Verbindung ab der 32. oder ab der 35. Schwangerschaftswoche vorsehen.

5. Bakterizide und/oder bakteriostatische Vaginalformulierung zur Verwendung in der Vorbeugung und in der Behandlung von bakteriellen Infektionen des Vaginaltrakts, umfassend mindestens eine Verbindung nach Anspruch 1, gegebenenfalls in Verbindung mit einem Wirkstoff, ausgewählt aus Laurinsäure, Monoglycerolester von Laurinsäure und Mischungen davon.

6. Bakterizide und/oder bakteriostatische Vaginalformulierung zur Verwendung nach Anspruch 5, wobei die Verbindung, die ausgewählt ist aus Caprylyl/Capryl-Glucosid, Lauryl-Glucosid, C8-C10-Alkyl-Polyglucosid, C12-C16-Alkyl-Polyglucosid und Mischungen davon, und der Wirkstoff, der ausgewählt ist aus Laurinsäure, Monoglycerolester von Laurinsäure und Mischungen davon, bevorzugt in einem Verhältnis vorliegen, das zwischen 1:10 und 10:1 umfasst ist.

7. Bakterizide und/oder bakteriostatische Vaginalformulierung zur Verwendung nach Anspruch 5 oder 6, wobei die Formulierung ein Vaginalgel, eine Vaginalspülung, eine Vaginalcreme, Salbe, Vaginalschaum, Vaginaltabletten, Hart- und Weichvaginalkapseln ist.

## Revendications

1. Composé choisi parmi un caprylyl/caprylglucoside, un lauryl-glucoside, un C8-C10 alkylpolyglucoside, un C12-C16 alkylpolyglucoside et leurs mélanges destiné à être utilisé comme agent bactériostatique ou bactéricide dans la prévention et dans le traitement des infections bactériennes des voies vaginales.

2. Composé selon la revendication 1 destiné à être utilisé selon la revendication 1, où lesdites infections bactériennes des voies vaginales comprennent des infections par *Gardnerella vaginalis, Neisseria gonorrheae, Atopobium vaginae, Chlamidia trachomatis, Trichomonas vaginalis, Mycoplasma genitalium*/*urealiticum*/*hominis*/*parvum, Treponema pallium* et *Streptococcus agalactiae.*

3. Composé selon la revendication 1 destiné à être utilisé selon la revendication 1, où lesdites infections bactériennes des voies vaginales sont des infections par *Streptococcus agalactiae.*

4. Composé selon l'une quelconque des revendications 1 à 3 destiné à être utilisé selon la revendication 1, où ladite prévention et/ou ledit traitement prévoient l'administration dudit composé à partir de la 32^{ème} ou à partir de la 35^{ème} semaine de grossesse.

5. Formulation vaginale bactéricide et/ou bactériostatique destinée à être utilisée dans la prévention et dans le traitement des infections bactériennes des voies vaginales, comprenant au moins un composé selon la revendication 1, éventuellement en association avec un ingrédient actif choisi parmi l'acide laurique, un monoester de glycérol et d'acide laurique et leurs mélanges.

6. Formulation vaginale bactéricide et/ou bactériostatique destinée à être utilisée selon la revendication 5, où ledit composé choisi parmi un caprylyl/caprylglucoside, un laurylglucoside, un C8-C10 alkylpolyglucoside, un C12-C16 alkylpolyglucoside et leurs mélanges et ledit ingrédient actif choisi parmi l'acide laurique, un monoester de glycérol et d'acide laurique et leurs mélanges sont de préférence dans un rapport compris entre 1:10 et 10:1.

7. Formulation vaginale bactéricide et/ou bactériostatique destinée à être utilisée selon la revendication 5 ou 6, où ladite formulation est un gel vaginal, un lavage vaginal, une crème vaginale, une pommade, une mousse vaginale, des comprimés vaginaux, des capsules vaginales dures et molles.
